(19) **Europäisches Patentamt / European Patent Office / Office européen des brevets**

(11) **EP 4 782 021 A1**

(12) **EUROPEAN PATENT APPLICATION**
published in accordance with Art. 153(4) EPC

(43) Date of publication:
29.07.2026 Bulletin 2026/31

(21) Application number: 24880215.9

(22) Date of filing: 21.10.2024

(51) International Patent Classification (IPC):
*A61L 27/56* (2006.01)     *A61L 27/58* (2006.01)
*A61L 27/18* (2006.01)

(52) Cooperative Patent Classification (CPC):
A61L 27/18; A61L 27/56; A61L 27/58

(86) International application number:
PCT/KR2024/016005

(87) International publication number:
WO 2025/084878 (24.04.2025 Gazette 2025/17)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 20.10.2023 KR 20230141413

(71) Applicant: Celloid Co., Ltd.
Pohang-si, Gyeongsangbuk-do 37673 (KR)

(72) Inventors:
• LEE, Sung Jin
  Pohang-si, Gyeongsangbuk-do 37673 (KR)
• LEE, Dong Hyeon
  Pohang-si, Gyeongsangbuk-do 37673 (KR)
• PARK, Mina
  Pohang-si, Gyeongsangbuk-do 37673 (KR)
• SONG, Jennifer Kim
  Pohang-si, Gyeongsangbuk-do 37673 (KR)

(74) Representative: BCKIP Part mbB
Landsberger Straße 98
80339 München (DE)

(54) **POROUS MEMBRANE AND METHOD FOR MANUFACTURING SAME**

(57) The present invention relates to a porous membrane and a method for manufacturing same and more specifically, to a membrane usable for implant materials, wound-covering materials, porous meshes, and the like, and to a method for manufacturing same.

Fig.8

EP 4 782 021 A1

## Description

### Technical Field

The present invention relates to porous membrane and a method for manufacturing the same, and more specifically, to a membrane usable for implant materials, wound-covering materials, porous meshes, and the like; and a method for manufacturing the same.

### Background Art

A conventional membrane has a problem in that surface unevenness and protrusions are generated as the thickness increases. Such unevenness and protrusions hinder uniform adhesion in close contact with a lesion when the membrane is used as an implant material, and in severe cases, may promote fibrosis or scar formation, thereby aggravating the lesion.

The present invention has been made in view of the above-described problems, and relates to a method for manufacturing a membrane in which unevenness or protrusions are minimized or absent even when the thickness of the membrane is increased, and to structural characteristics of the membrane manufactured thereby.

In addition, the present invention has been made to provide additional technical features that cannot be easily conceived by those skilled in the art, in addition to the above-described technical concept.

### Disclosure

### Technical Problem

Accordingly, the present invention has been made in view of the above-mentioned problems occurring in the related art, and it is an objective of the present invention to provide a method for manufacturing a membrane in which unevenness or protrusions are not generated even though a thickness increases.

It is another objective of the present invention to provide a porous membrane having a thickness applicable to implant materials, wound-covering materials, and the like, and having a structure of a flat surface.

Objectives of the present invention are not limited to the objectives described above, and other objectives that are not described will be clearly understood by a person skilled in the art from the description below.

### Technical Solution

To accomplish the above-mentioned objectives, a porous membrane according to the present invention has a thickness of at least 500 $\mu$m, and a roughness less than a preset value, wherein the roughness is defined as a ratio of a height of a protrusion protruding from a plane relative to the thickness of the membrane, and the preset value is 1.

Moreover, a porous membrane according to another embodiment of the present invention has a thickness of at least 500 $\mu$m and an evenness greater than a preset value, wherein the evenness is defined as a reciprocal of an area ratio of one or more protrusions relative to an area of the membrane, and the preset value is 50. In this case, the protrusion is defined as a structure having a height of at least 200 $\mu$m from a plane of the membrane, or a structure in which a ratio of a height of the protrusion from the plane of the membrane to the thickness of the membrane is 1 or more. Moreover, a total area of the protrusion(s) is defined as a total area of black regions obtained by converting a membrane image into black and white, wherein the membrane image is obtained by capturing the membrane on a surface light source.

Meanwhile, a porous membrane according to a further embodiment of the present invention has a thickness of at least 500 $\mu$m and a uniformity greater than a preset value, wherein the uniformity is defined as a reciprocal of a difference between a first contrast ratio corresponding to a first reference region on the membrane and a second contrast ratio corresponding to a second reference region that does not overlap the first reference region, and the preset value is 0.1.

A method for manufacturing a porous membrane according to another embodiment of the present invention may include: grounding a collector having a surface; electrospinning nanofibers in a charged state onto the surface of the collector; and applying an electrolyte to the nanofibers stacked on the surface of the collector.

Furthermore, in the method for manufacturing the porous membrane, the step of electrospinning of nanofibers may include stacking the nanofibers on the surface of the collector between a first point and a second point by controlling a nozzle (needle) for electrospinning the nanofibers to repeatedly reciprocate toward the first point and the second point.

Additionally, in the method for manufacturing the porous membrane, the applying of the electrolyte is periodically performed whenever reciprocating motion of a nozzle is performed a predetermined number of times.

In addition, the method for manufacturing the porous membrane may further include peeling the nanofibers stacked on the surface of the collector, wherein the peeling of the nanofibers includes attaching a frame corresponding to a region to be peeled on the surface of the collector, and cutting the nanofibers along the frame after the frame is attached.

**Advantageous Effect**

According to the present invention, it is possible to obtain a membrane having a thickness sufficient for commercialization, for example, a thickness of at least 500 μm, while preventing formation of protrusions and maintaining a uniform structure without aggregation throughout an entire area.

Thus, the membrane obtained has a uniform surface despite its large thickness, exhibits excellent mechanical strength, and has high cell compatibility, and thus may be effectively used in medical fields such as artificial skin and wound-covering materials.

The effects of the present invention are not limited to those described above, and other effects not mentioned herein will be clearly understood by those skilled in the art from the following description.

**Description of Drawings**

FIG. 1 illustrates a configuration of an apparatus for preparing a membrane.

FIGs. 2a to 2e illustrate enlarged photographs of surfaces of membranes manufactured with different K values.

FIG. 3a to 3d illustrate graphs for facilitating understanding of characteristics of the membrane.

FIG. 4 illustrates a process of converting a membrane image into black and white and a result thereof.

FIGS. 5a to 7b illustrate states in which contrast ratios are compared by setting two reference regions in each membrane when K is 2000, 6000, and 10000.

FIG. 8 illustrates a sequence of a method for manufacturing a membrane according to the present invention.

FIG. 9 illustrates a photograph of the membrane according to the present invention taken from a side.

FIG. 10 illustrates a diagram for facilitating understanding of roughness as a first characteristic value.

FIG. 11 illustrates a diagram for facilitating understanding of evenness as a second characteristic value.

FIG. 12 illustrates a diagram for facilitating understanding of uniformity as a third characteristic value.

FIGs. 13a to 13d illustrates an experimental state for confirming flexibility of the membrane according to the present invention.

**Mode for Invention**

Hereinafter, embodiments of the present invention will be described in detail with reference to the accompanying drawings. The advantages and features of the present invention, and methods for accomplishing the same, will become apparent from the following description of embodiments with reference to the accompanying drawings. However, the present invention is not limited to the embodiments disclosed herein, but may be implemented in various forms. The embodiments are provided so that the present invention will be fully disclosed and will fully convey the scope of the invention to those skilled in the art, and the present invention is defined only by the scope of the appended claims. Like reference numerals refer to like elements throughout the specification.

Unless otherwise defined, all terms (including technical and scientific terms) used herein have meanings commonly understood by those skilled in the art to which the present invention pertains. In addition, terms defined in commonly used dictionaries should not be interpreted in an idealized or overly formal sense unless expressly so defined herein. The terms used herein are for the purpose of describing embodiments and are not intended to limit the present invention. As used herein, a singular form includes plural forms unless the context clearly indicates otherwise.

Terms such as "first" and "second" are used only to distinguish one component from another component, and do not limit the scope of the present invention. For example, a first component may be referred to as a second component, and similarly, a second component may be referred to as a first component.

As used herein, the terms "comprise" and/or "comprising" do not preclude the presence or addition of one or more other elements, steps, operations, and/or components.

First, in order to more accurately understand a conventional membrane and a membrane according to the present invention, basic steps of a method for manufacturing a membrane and a manufacturing environment will be described.

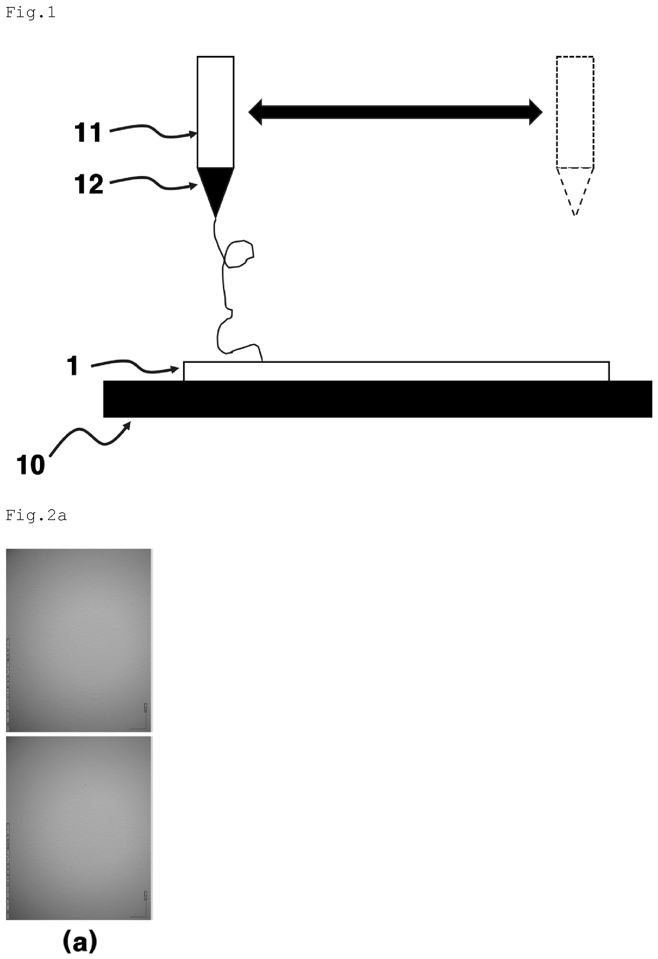

FIG. 1 illustrates a configuration of a manufacturing apparatus required for manufacturing a membrane. Referring to the drawing, the manufacturing apparatus mainly includes a collector 10 on which electrospun nanofibers are stacked, a syringe 11 supplying a polymer solution at a constant pressure, and a nozzle 12 for electrospinning the polymer solution supplied through the syringe 11 in a state where a high voltage is applied. Although not illustrated, the manufacturing apparatus may further include a power supply for applying the high voltage and a control unit for controlling overall components. Moreover, the manufacturing apparatus may be controlled such that the nozzle 12 or a plurality of nozzles 12 perform repeated horizontal reciprocating motion at a position spaced apart from the collector 10 arranged on the bottom surface by a predetermined distance, and nanofibers may be stacked on the collector 10 through the reciprocating motion. In the present invention, the manufacturing apparatus includes ten syringes 11 and ten nozzles 12 arranged in a line in one direction, and the syringes 11 and the nozzles 12 are controlled to perform reciprocating motion in a direction perpendicular to an arrangement direction thereof.

Unless other control variables are changed, a thickness of nanofibers stacked on the collector 10 is known to be proportional to the number of reciprocations of the nozzle 12. In experimental examples to be described later, it will be examined what thickness values the membrane has according to the number of movements of the nozzle 12, and how many protrusions are formed on the surface as the number of movements increases, that is, as the thickness of the membrane increases. For convenience of explanation, K is used to denote the number of movements. For example, when the nozzle 12 moves from a first position to a second position, K has a value of 1, and when the nozzle returns from the second position to the first position, K has a value of 2. That is, when the nozzle 12 performs one reciprocation, K has a value of 2.

Meanwhile, the polymer solution may be prepared using various combinations of solvents and solutes. In particular, examples of the solute may include polycaprolactone (PCL), polyethylene oxide (PEO), polyvinyl alcohol (PVA), polyacrylonitrile (PAN), polysulfone (PSf), polyethersulfone (PES), polyvinylidene fluoride (PVDF), chitosan, and silk fibroin. The nanofibers referred to in the present detailed description were prepared using 1,1,1,3,3,3-hexafluoro-2-propanol as a solvent, polycaprolactone at 4.5 wt% as a first solute, and poly(ethylene glycol)-block-poly(propylene glycol)-block-poly (ethylene glycol) at 4.5 wt% as a second solute. However, it should be understood that combinations of solvents and solutes of the polymer solution capable of electrospinning are not limited to the above example. In experimental examples to be described later, based on a state in which the nozzle 12 moved 2000 times, a discharge amount per syringe was 1.436 ml, and considering that a total of 10 syringes were used, it was confirmed that 14.36 ml of the polymer solution was discharged. In addition, a discharge flow rate was 1.5 ml/hr. Meanwhile, it should be understood that a diameter of the nanofibers referred to in the present detailed description is controllable within a range of 100 nm to 999 nm.

As described above, the method for manufacturing a membrane according to the conventional method and the manufacturing apparatus have been described.

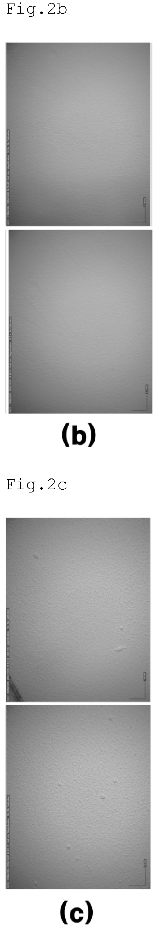

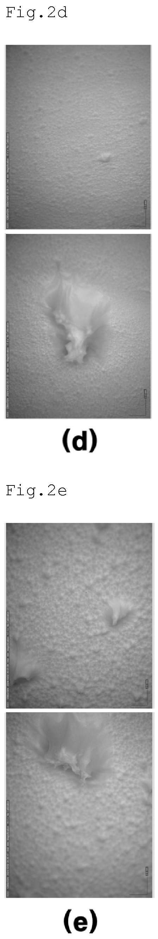

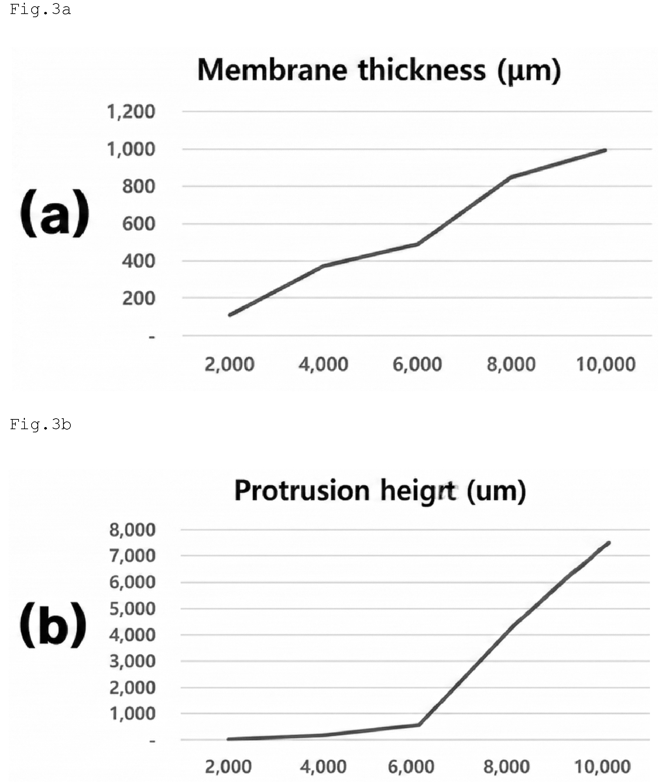

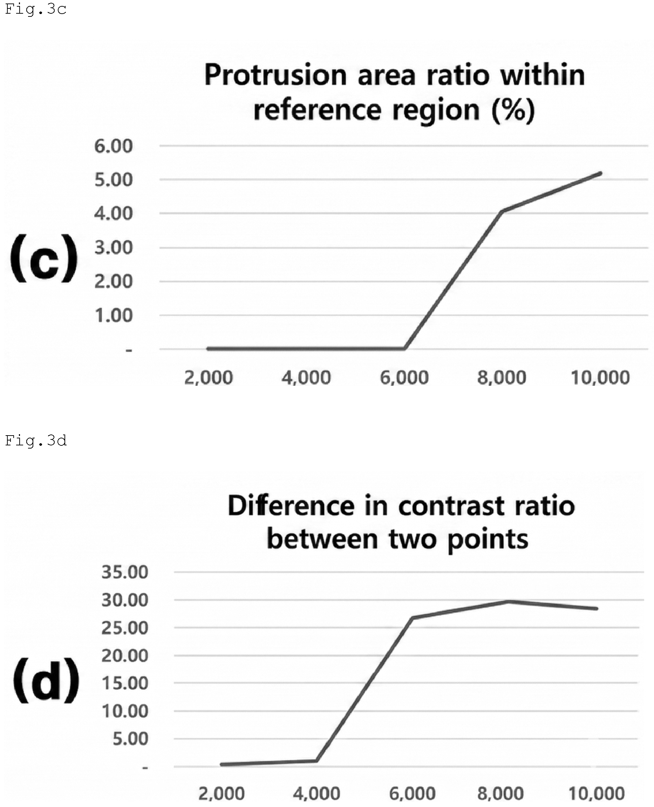

FIG. 2 illustrates photographs of surfaces of membranes manufactured according to a conventional method, Table 1 below shows a comparison of several characteristics obtained by analyzing the membranes generated at K values of 2000, 4000, 6000, 8000, and 10000, and FIG. 3 illustrates graphs representing the characteristics recorded in Table 1. Before describing in detail the membrane according to the present invention and a method for manufacturing the same, characteristics of membranes manufactured according to the conventional method, particularly those of membranes manufactured to have a thickness greater than or equal to a predetermined level, will be described first.

Meanwhile, in Table 1, it was confirmed that the thickness of the membrane increased as the K value increased, as expected, and a linear relationship was also confirmed in graph (a) of FIG. 3.

[Table 1]

| K | 2,000 | 4,000 | 6,000 | 8,000 | 10,000 |
|---|---|---|---|---|---|
| Membrane thickness ($\mu$m) | 111 | 373 | 489 | 851 | 992 |
| Protrusion height ($\mu$m) | - | 167 | 556 | 4,365 | 7,503 |
| Area ratio of protrusions within reference region | - | - | 0.02 | 4.06 | 5.18 |
| Difference in contrast ratio between two arbitrary points | 0.41 | 1.08 | 26.82 | 29.66 | 28.41 |

FIG. 2 shows images of surfaces of membranes photographed at a magnification of 17 times, and a scale bar in each image is 2 mm. Referring to the drawings, at K=2000 and K=4000, no protrusions visible to the naked eye or other heterogeneous structures were observed on the membrane surface, whereas at K=6000, K=8000, and K=10000, distinguishable protrusions were observed. At K=6000, small-scale protrusions were sparsely present within a reference area, whereas at K≥8000, large-scale protrusions were present in large numbers while occupying a wide area.

The tendency observed in FIG. 2 was also quantitatively confirmed through Table 1 and FIG. 3.

First, as a result of measuring heights of arbitrary protrusions observed when the manufactured membrane was viewed from a side, no protrusion was observed at K=2000 and thus measurement was not possible, and protrusion heights of 167 $\mu$m at K=4000, 556 $\mu$m at K=6000, 4365 $\mu$m at K=8000, and 7503 $\mu$m at K=10000 were measured. When expressed as a graph, as shown in the graph of FIG. 3(b), it was confirmed that up to K=6000, that is, until the thickness of the membrane reached 489 $\mu$m, protrusions were hardly observed or only protrusions having very small heights were formed, whereas in a process of forming a thicker membrane, a height, that is, a size of protrusions, increased rapidly.

Second, as a result of analyzing a ratio of an area occupied by one or more protrusions identified by a specific method within a predetermined area (reference area) of each membrane, no protrusions were identified at K=2000 and K=4000 and thus values could not be calculated, and protrusion area ratios of 0.02% at K=6000, 4.06% at K=8000, and 5.18% at K=10000 were obtained. When expressed as a graph, as shown in a graph of FIG. 3(c), it was confirmed that protrusions were hardly detected within the reference area up to K=6000, whereas in membranes manufactured to be thicker than this, an area occupied by protrusions increased rapidly.

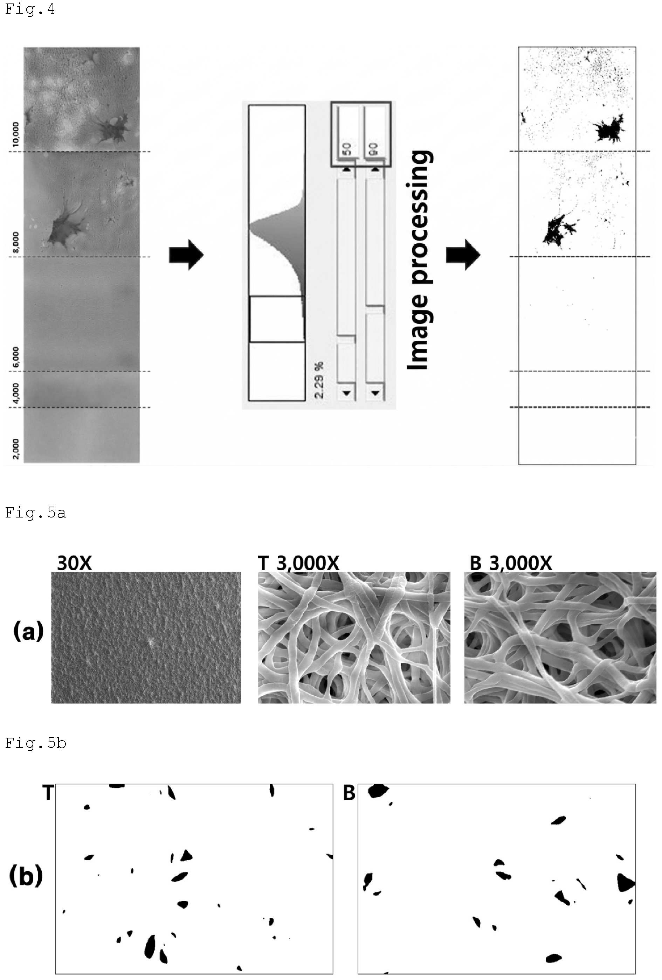

For reference, a method for identifying protrusions in the corresponding experiment will be briefly described as follows. First, a membrane having a reference area of a predetermined size is placed on a surface light source (a LUXPAD K22H product is used, and illuminance is set to 480 lux), and a first membrane image is obtained by capturing the membrane from a planar view. Thereafter, a second membrane image was obtained by performing image processing, more specifically, conversion into a black-and-white image (binarization), on the obtained first membrane image. Assuming that white is represented by 255, black is represented by 1, and values therebetween are represented by different shades of gray, the image processing in the present experiment was performed under a condition in which a threshold was set such that only pixels having values from 50 to 90 in the image were represented as black. FIG. 4 illustrates the processes in sequence, wherein a top image in FIG. 4 corresponds to the first membrane image and a bottom image corresponds to the second membrane image after the image processing. An area of protrusions within the reference area was calculated by assuming that the area is equal to an area occupied by black regions in the second membrane image.

Third, two reference regions were arbitrarily selected on the surface of each membrane, and a difference between contrast ratios of the respective reference regions obtained through image analysis was analyzed. In this instance, the contrast ratio refers, more specifically, to a contrast ratio on the membrane surface that reflects a degree of entanglement, that is, a density, of nanofibers. As a result of obtaining contrast ratios from the two reference regions for each membrane and analyzing a difference therebetween, values of 0.41 at K=2000, 1.08 at K=4000, 26.82 at K=6000, 29.66 at K=8000, and 28.41 at K=10000 were obtained, and when such characteristics were expressed as a graph, a tendency as shown in FIG. 3(d) was observed.

Meanwhile, in the experiment, when selecting the reference regions, based on observation of SEM images, a region in which protrusions were present was defined as a first reference region (T), and a planar region in which protrusions were not present was defined as a second reference region (B). The contrast ratio in each reference region refers to a value expressed as a percentage obtained by dividing an area occupied by black regions by an area occupied by white regions within the reference region when the SEM image is converted into a black-and-white image (using an Image J program). When performing the conversion into the black-and-white image, there is no particular limitation on a threshold setting as long as a degree of entanglement of nanofibers can be identified from the SEM image. However, when comparing two reference regions within at least one membrane, it is preferable that the threshold setting is consistently maintained so that the contrast ratio of the first reference region and the contrast ratio of the second reference region are obtained.

[Equation 1]

$$Contrast\ Ratio = \frac{Black\ Area}{White\ Area} \times 100$$

As a result of calculating contrast ratios for the respective reference regions according to Equation 1, in the reference region (T) including protrusions, particularly in a thick membrane, nanofibers no longer exhibited a normal fibrous structure, that is, a woven structure, but instead exhibited a collapsed structure, such that black regions were scarcely observed. In contrast, in a planar reference region (B) in which no protrusions or heterogeneous structures were present, particularly in a thin membrane, nanofibers exhibited a normal woven structure, and black regions were observed in an SEM image due to pores formed in the woven structure.

FIGS. 5 to 7 illustrate states in which contrast ratios were compared by selecting two reference regions in each membrane at K=2000, K=6000, and K=10000, respectively. Referring to FIG. 5, FIG. 5(a) illustrates a protrusion observed in an SEM image (at a magnification of 30 times) of a membrane surface at K=2000, an SEM image magnified 3000 times of a reference region including the protrusion (the first reference region), and an SEM image magnified 3000 times of the reference region in which no protrusion is present (the second reference region). FIG. 5(b) illustrates results obtained by performing image processing on the first reference region and the second reference region. From the images, the contrast ratio of the first reference region was 1.63% and that of the second reference region was 2.04%, and the difference therebetween, 0.41, corresponds to the contrast ratio difference at K=2000 described in Table 1.

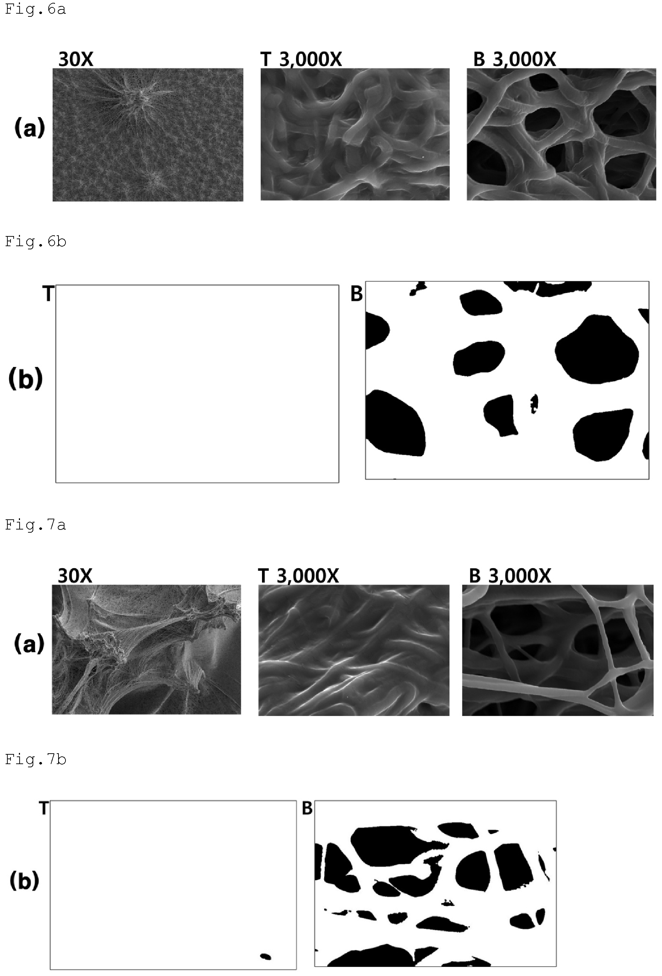

Similarly, FIG. 6(a) illustrates a protrusion observed in a SEM image magnified 3000 times, and SEM images magnified 3000 times of a reference region including the protrusion (first reference region) and a reference region in which no protrusion is present (second reference region). FIG. 6(b) illustrates results obtained by performing image processing for calculating contrast ratios of the respective reference regions. Notably, when K is 6000, that is, when the membrane has a thickness of 489 $\mu$m, black regions are hardly observed in an enlarged image of the first reference region, and thus a contrast ratio is calculated as 0.00%, whereas in an enlarged image of the second reference region, a contrast ratio is calculated as 26.82%. As confirmed through the SEM images, when the membrane is manufactured to have a thickness greater than or equal to a predetermined level, protrusions no longer exhibit characteristics of a woven structure of stacked nanofibers, and thus, it may be inferred that the corresponding reference region of the membrane loses functions that a porous structure may have.

Likewise, FIG. 7(a) illustrates a protrusion observed in an SEM image of a membrane at K=10000 magnified 30 times, and SEM images of a reference region including the protrusion (the first reference region) and a reference region in which no protrusion is present (the second reference region), each magnified 3000 times. FIG. 7(b) illustrates results obtained after image processing of the first reference region and the second reference region. The membrane at K=10000 exhibited analysis results similar to those described with reference to FIG. 6. The contrast ratio of the first reference region was 0.12%, the contrast ratio of the second reference region was 28.53%, and the difference between the two reference regions was 28.41.

From a comparison of contrast ratios in the two reference regions of the membrane, it was found that, as the thickness of the membrane increased, protrusions or heterogeneous structures, rather than a normal fibrous structure, were formed more frequently. As a result, the contrast ratio in the first reference region became very small, leading to an increase in the difference in contrast ratio between the first reference region and the second reference region.

As described above with reference to FIGS. 2 to 7, characteristics of membranes manufactured according to the conventional method, particularly thick membranes having K of 6000 or more or a thickness of about 500 $\mu$m or more, have been described. In summary, compared to membranes having a smaller thickness, the membranes having K of 6000 or more or a thickness of about 500 $\mu$m or more exhibit a problem in that a height of the protrusions increases more rapidly, resulting in formation of protrusions at a large scale. In addition, when considering an area ratio of protrusions within a predetermined reference region, the membranes having K of 6000 or more or a thickness of about 500 $\mu$m or more showed a rapidly increasing tendency, indicating that protrusions or heterogeneous structures are formed over a wide area. Moreover, when analyzed based on a difference in contrast ratio between two regions, it is confirmed that, when the membrane is manufactured to be thicker than a membrane having K of 4000 or more or a thickness of about 400 $\mu$m, a normal woven structure is not formed due to protrusions or heterogeneous structures.

As described above, when a membrane is manufactured according to the conventional method, structural characteristics that may adversely affect the quality of the membrane appear as the thickness of the membrane increases. Such characteristics may hinder a smooth supply of nutrients or oxygen and may also prevent the membrane from being uniformly adhered to a lesion, thereby acting as obstacles to commercialization of the membrane.

The membrane according to the present invention is improved such that the above-described characteristics do not occur. Hereinafter, structural characteristics of the membrane according to the present invention will be described with reference to several verifiable parameters, and a method for manufacturing the membrane will also be described in detail.

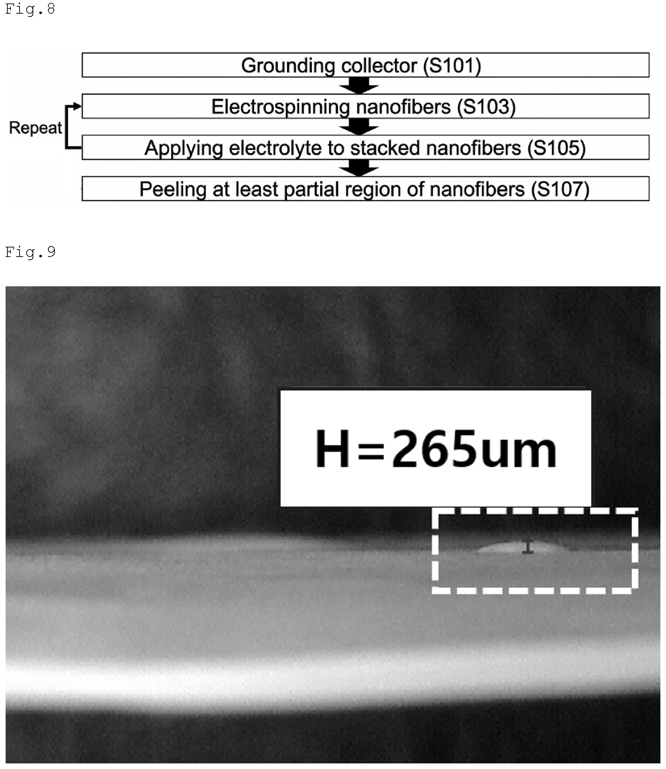

FIG. 8 illustrates a method for manufacturing a membrane according to the present invention in sequence. As described above with reference to FIG. 1, the same manufacturing apparatus as that described with reference to FIG. 1 is used in the method for manufacturing the membrane according to the present invention. However, the manufacturing method according to the present invention is characterized in that a step of applying an electrolyte solution is further included during a process of stacking nanofibers.

Referring to the drawings, the method for manufacturing the membrane according to the present invention starts with grounding a collector 10 (S101), and then includes electrospinning nanofibers (S103), applying an electrolyte to nanofibers stacked on the collector 10 (S105), and peeling off at least a portion of the nanofibers from the collector 10 (S107). The step S101 and the step S103 are substantially the same as those of the conventional method for manufacturing a membrane, whereas the step S105 is newly introduced in the present invention. The electrolyte neutralizes charges of the nanofibers stacked on the collector 10 during an electrospinning process, thereby reducing electrostatic repulsive force. Specifically, the electrospinning of nanofibers in the step S103 is a process of generating nanofibers by applying a high voltage to a polymer solution. In this case, the polymer solution becomes electrically charged, and the generated nanofibers also carry charges. Accordingly, when the nanofibers are stacked on the collector 10, electrostatic repulsive force is generated between the charges. In particular, as the thickness of the membrane increases, that is, as the amount of nanofibers stacked on the collector 10 increases, the electrostatic repulsive force becomes stronger. As a result, the nanofibers are not uniformly stacked on the surface of the collector 10 but become aggregated or entangled, thereby causing formation of protrusions or heterogeneous structures on the membrane surface. The step S105 is a step of neutralizing the charges by applying the electrolyte to the stacked nanofibers. When the electrolyte comes into contact with the nanofibers, the charges of the nanofibers are neutralized, thereby reducing the electrostatic repulsive force. As the electrostatic repulsive force decreases, the nanofibers can be uniformly stacked on the surface of the collector 10, thereby minimizing formation of protrusions on the membrane surface and enabling manufacture of a high-quality membrane having a large thickness and a uniform surface. Meanwhile, a type of electrolyte usable in the step S105 is not particularly limited. As a solvent of the electrolyte solution, water, ethanol, or methanol may be used, and as a solute, various salts such as sodium chloride (NaCl), potassium chloride (KCl), and calcium chloride (CaCl2) may be used.

On the other hand, the step S105 may be repeatedly performed whenever electrospinning of nanofibers proceeds for a predetermined period of time, that is, whenever the nanofibers are stacked on the collector 10 to a predetermined thickness. Accordingly, whenever the stacked nanofibers reach a certain thickness, charge neutralization may be performed so that stacking of nanofibers up to a subsequent thickness may proceed smoothly. For example, the electrolyte may be applied to the previously stacked nanofibers whenever the K value reaches 2000, and by repeating this process several times, a membrane having a desired thickness may be manufactured. Preferably, the step of applying the electrolyte may be performed whenever the K value reaches a value between 2000 and 3000.

For reference, it is preferable that electrospinning of nanofibers is not stopped while the step S105 of applying the electrolyte is being performed, that is, electrospinning of the nanofibers is continuously maintained. However, measures may be taken such that the electrospun nanofibers are not directed toward the collector 10. For example, while the step S105 is being performed, a separate metal plate may be disposed in front of the nozzle tip so that the nanofibers are not directed toward the collector 10. This is because, when electrospinning of the nanofibers is stopped and then resumed, electrospinning characteristics of nanofibers newly electrospun through the nozzle may change, thereby affecting uniformity of the membrane.

Meanwhile, a method of applying the electrolyte to the nanofibers is not particularly limited. Preferably, a dipping method in which the nanofibers are immersed in an electrolyte solution, or a spraying method in which the electrolyte is applied to the nanofibers by spraying, may be used. The dipping method may be realized in various ways, such as a method in which the collector 10 is detached after the nanofibers are stacked on the collector 10 and then immersed in an electrolyte container, or a method in which the collector 10 is configured to be immersed in the electrolyte container within the membrane manufacturing apparatus according to a control command. The dipping method is a method capable of most reliably neutralizing charges of the nanofibers. The spraying method is a method in which the electrolyte solution is sprayed onto the nanofibers on the collector 10 under predetermined conditions using a spraying means capable of spraying the electrolyte solution. The electrolyte solution may be sprayed by an operator using a tool, or may be sprayed by an automatic spraying configuration provided in the membrane manufacturing apparatus. Although the dipping method is more effective for charge neutralization than the spraying method, either of the two methods may be selectively used as necessary, and both methods may also be used in combination as needed.

Meanwhile, the step S107 is a step of peeling the nanofibers from the collector 10 to finally obtain the membrane. In this step, a peeling frame may be used to effectively peel the nanofibers. The frame made of metal, plastic, or the like may be placed on the nanofibers after stacking is completed, and the membrane may be cut and peeled along a shape of the frame by a cutting means held by an operator or by an automatically controlled cutting means.

As described above with reference to FIG. 8, the method for manufacturing the membrane according to the present invention has been described. Hereinafter, structural characteristics of the membrane according to the present invention will be described.

FIG. 9 illustrates a photograph of a membrane according to the present invention taken from a side. The photograph shows a membrane obtained by stacking nanofibers until K reaches 10000 and then peeling off the nanofibers using the above-described method, that is, the method further including the step of applying an electrolyte. The membrane has a thickness of 1067.8 $\mu$m, and a height of protrusions observed on a surface of the membrane is measured as 265 $\mu$m. Compared with the membrane manufactured according to the conventional method at K=10000 as shown in Table 1, which has a thickness of 992 $\mu$m and a protrusion height of as much as 7503 $\mu$m, the membrane according to the present invention has a significantly greater thickness while exhibiting very small protrusions, and thus it can be confirmed that the surface of the membrane is overall formed to be flat.

As described above, the membrane according to the present invention has a large thickness and a uniform surface and a uniform internal structure. The membrane manufactured may be defined by several characteristic values as follows. It should be noted that the membrane according to the present invention is premised on having a thickness of 500 $\mu$m or more. It may be understood as a condition based on the fact, as described above with reference to Table 1, that in the conventional method, heterogeneous structures such as protrusions appear when the membrane has a thickness of 500 $\mu$m or more. That is, since it is not possible to manufacture a membrane having a thickness of 500 $\mu$m or more and maintaining a uniform surface and a uniform internal fibrous structure according to the conventional method, the membrane according to the present invention is defined as having a thickness of 500 $\mu$m or more.

First, the membrane according to the present invention may be defined by roughness (a first characteristic value). The roughness may be defined as a ratio of a height of a protrusion formed to protrude from a plane of the membrane relative to the thickness of the membrane, and the roughness is required to have a value less than a preset value of 1. FIG.

10 illustrates a diagram for facilitating understanding of the roughness characteristic value. For reference, a plurality of protrusions may be formed on the surface of the membrane. In this case, the protrusion used for calculating the roughness may be determined as one having the greatest height among them. Alternatively, when a plurality of protrusions exist, an average value of heights of the protrusions may be determined as a protrusion height value so that the roughness is calculated based thereon.

On the other hand, limiting the roughness to have a value less than 1 is based on the experimental results described above. Table 2 below shows values of roughness, that is, ratios of protrusion height to membrane thickness, calculated for membranes having K values from 2000 to 10000. Referring to the table, when K is 6000, that is, when the membrane thickness approaches 500 $\mu$m and protrusions on the surface are formed at an acceptable level, the roughness is calculated as 1.137. Based on the above, the membrane intended to be implemented in the present invention is defined as having a thickness of at least 500 $\mu$m and a roughness less than 1.

[Table 2]

| K | 2,000 | 4,000 | 6,000 | 8,000 | 10,000 |
|---|---|---|---|---|---|
| Membrane thickness ($\mu$m) | 111 | 373 | 489 | 851 | 992 |
| Protrusion height ($\mu$m) | - | 167 | 556 | 4,365 | 7,503 |
| Ratio of protrusion height to thickness (r) | 0 | 0.447721 | 1.137014 | 5.12926 | 7.563508 |

Meanwhile, in order to calculate the above characteristic value, it is necessary to accurately determine a thickness (D) of the membrane and a height (h) of the protrusion. The thickness may be determined as an average value of values obtained by measuring thicknesses at a plurality of points (e.g., at least 20 or more points) on the membrane, or may be determined based on a height calculated with reference to a known area of the membrane (the area is determined when the membrane is peeled in a state where a frame is attached), a density of a material, and a measured weight, or may be determined as a value measured from an image obtained by photographing the membrane placed on a smooth surface such as a metal surface from a side. Furthermore, the height (h) of the protrusion may be determined as a length exceeding the thickness value from a bottom surface of the membrane once the thickness (D) of the membrane is determined, or may be determined as a value measured from an image obtained by photographing the protrusion from a side.

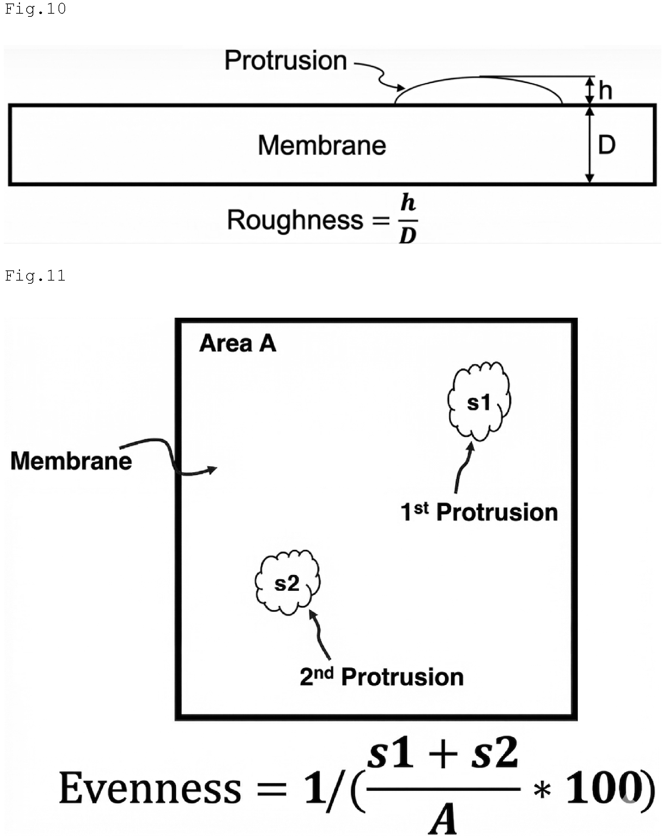

Second, the membrane according to the present invention may also be defined by evenness (a second characteristic value). The evenness is defined as a reciprocal of an area ratio of protrusion(s) relative to a reference area of the membrane when viewed from a plane, and the evenness is required to have a value greater than a preset value of 50. FIG. 11 illustrates how evenness is calculated when protrusions having areas s1 and s2 exist on a membrane having an area A.

The limitation that the evenness has a value greater than 50 is based on the above-described experimental results. Table 3 below shows results of calculating area ratios of protrusions (%) within a reference region, and evenness values calculated by taking reciprocals thereof. For reference, in the previous experimental results, protrusions are not observed when K is 2000 or 4000 as observed using a surface light source. However, in Table 3 below, estimated values of protrusion area ratios at K=2000 and K=4000 are arbitrarily provided based on K=6000. Referring to the table, when K is 6000, that is, when the thickness of the membrane approaches 500 $\mu$m, the protrusion area ratio within the reference region is 0.02%, and the evenness is 50. Based on the above, the membrane intended to be realized in the present invention is defined to have an evenness greater than that of the above experimental example.

[Table 3]

| K | 2,000 | 4,000 | 6,000 | 8,000 | 10,000 |
|---|---|---|---|---|---|
| Area ratio of protrusions within reference region (%) | 0.005 (est.) | 0.01 (est.) | 0.02 | 4.06 | 5.18 |
| Evenness | 200 | 100 | 50 | 0.25 | 0.19 |

Meanwhile, in the above experiment, when calculating the area ratio of protrusions (%) within the reference region, the membrane was placed on a surface light source and photographed, and the captured image was converted into black and white through image processing to obtain a ratio of an area occupied by black regions (regions identified as protrusions) within the reference region. However, methods for calculating the protrusion area ratio (%) are not limited to the above-described method, and various methods may be used. For example, when a membrane is imaged from a side at an enlarged scale, if a structure having a height of 200 $\mu$m or more is present on a surface of the membrane, all such structures may be regarded as protrusions, and a ratio of areas occupied by the protrusions on a plane may be calculated. Alternatively, all structures in which a ratio of a height protruding from the surface of the membrane to a thickness of the membrane is 1 or more may be regarded as protrusions, and a ratio of areas occupied by the protrusions may also be calculated.

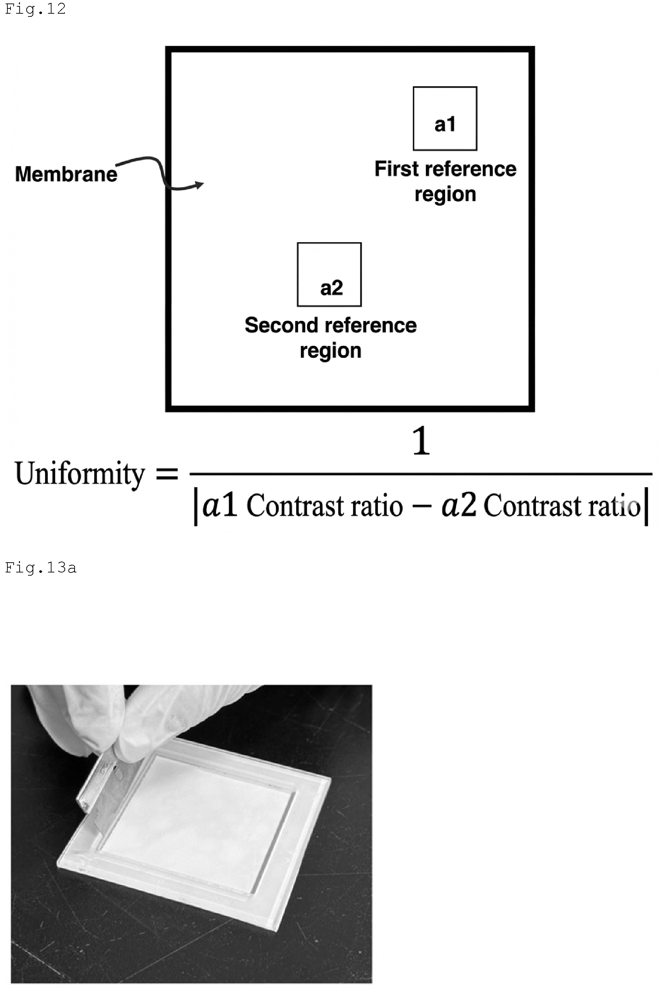

Third, the membrane according to the present invention may also be defined by uniformity (a third characteristic value). The uniformity is defined as a reciprocal of a difference between a first contrast ratio corresponding to a first reference region on the membrane and a second contrast ratio corresponding to a second reference region, and the uniformity is required to be greater than a preset value of 0.1. FIG. 12 illustrates how the uniformity is calculated when the first reference region (a1) and the second reference region (a2) are determined and contrast ratio values for the respective reference regions are obtained. The contrast ratio used for calculating the uniformity may be obtained in the same manner as described in the experimental examples with reference to FIGS. 4 to 7.

The limitation that the uniformity is greater than 0.1 is based on the experimental results described above. Table 4 below shows experimental results of calculating a difference in contrast ratio (%) between two arbitrary points on the membrane, and uniformity values obtained by taking reciprocals of the difference. It can be seen that the contrast ratio difference is 1.08 at K=4000, whereas it sharply increases to 26.82 at K=6000. Based on the experimental results, it may be inferred that the membrane has an acceptable level of uniformity when the value falls within a single-digit range. Accordingly, 0.1, which is obtained based on a contrast ratio difference value of 10, is set as the preset value.

[Table 4]

| K | 2,000 | 4,000 | 6,000 | 8,000 | 10,000 |
|---|---|---|---|---|---|
| Difference in contrast ratio between two arbitrary points (%) | 0.41 | 1.08 | 26.82 | 29.66 | 28.41 |
| Uniformity | 2.44 | 0.93 | 0.04 | 0.03 | 0.04 |

Meanwhile, the uniformity may be calculated only for the upper surface of the membrane. However, preferably, the uniformity may also be calculated for cross-sections obtained by cutting the membrane in a horizontal direction at a plurality of heights, so that it may be confirmed whether an internal portion of the membrane also has a normal fibrous structure. For example, when uniformity of a membrane having a thickness of 1000 $\mu$m is to be analyzed, the uniformity of cross-sections cut at intervals of 250 $\mu$m may be analyzed to determine whether the membrane satisfies conditions of the membrane according to the present invention.

Additionally, the uniformity is not limited to being analyzed based on only one set of reference regions (a first reference region and a second reference region), but may be calculated for multiple sets of reference regions (e.g., a third reference region and a fourth reference region, a fifth reference region and a sixth reference region, etc.), so that it may be determined whether the membrane to be analyzed satisfies conditions proposed in the present invention.

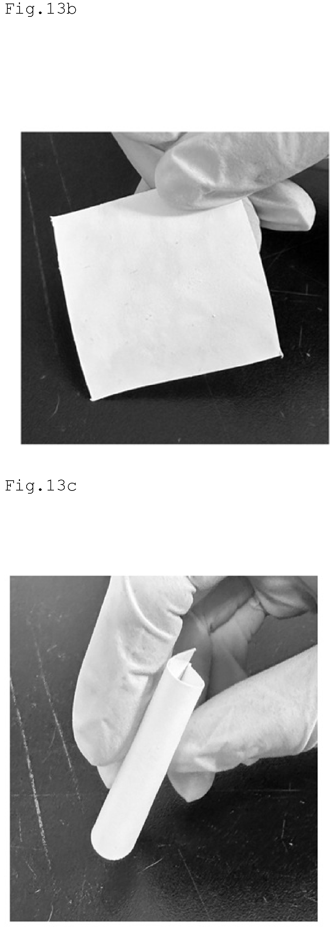

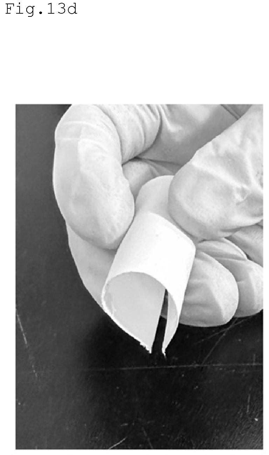

Meanwhile, FIGs. 13a through 13d illustrate a state in which flexibility of the membrane according to the present invention is confirmed. As shown in the drawing, when an external force is applied to the peeled membrane, the membrane may be bent without damage to a surface thereof, and thus, elasticity and flexibility of the membrane according to the present invention may be confirmed.

The present invention is not limited to the specific embodiments and examples described above, and various modifications and variations may be made by those skilled in the art without departing from the spirit and scope of the invention as defined in the appended claims. Such modifications and variations should not be understood as being separate from the technical idea and scope of the present invention.

**Claims**

1. A porous membrane comprising:

    a thickness of at least 500 $\mu$m, and
    a roughness less than a preset value,
    wherein the roughness is defined as a ratio of a height of a protrusion protruding from a plane of the membrane relative to the thickness of the membrane, and the preset value is 1.

2. The porous membrane according to claim 1, wherein the thickness of the membrane is calculated as an average value of a plurality of thickness values measured at a plurality of locations on the membrane.

3. A method for manufacturing a porous membrane, comprising:

    grounding a collector having a surface;

electrospinning nanofibers in a charged state onto the surface of the collector; and
applying an electrolyte to the nanofibers stacked on the surface of the collector.

4. The method for manufacturing a porous membrane according to claim 3, wherein the applying of the electrolyte is periodically performed whenever reciprocating motion of a nozzle is performed a predetermined number of times.

5. The method for manufacturing a porous membrane according to claim 3, further comprising:
peeling the nanofibers stacked on the surface of the collector.

EP 4 782 021 A1

Fig.1

Fig.2a

(a)

Fig.2b

**(b)**

Fig.2c

**(c)**

Fig.2d

**(d)**

Fig.2e

**(e)**

Fig.3a

**Membrane thickness (μm)**

(a)

Fig.3b

**Protrusion heigrt (um)**

(b)

Fig.3c

Protrusion area ratio within reference region (%)

(c)

Fig.3d

Diference in contrast ratio between two points

(d)

Fig.4

Image processing

Fig.5a

30X    T 3,000X    B 3,000X

(a)

Fig.5b

T    B

(b)

Fig.6a

Fig.6b

Fig.7a

Fig.7b

Fig.8

| Grounding collector (S101) |
| Electrospinning nanofibers (S103) |
| Applying electrolyte to stacked nanofibers (S105) |
| Peeling at least partial region of nanofibers (S107) |

Repeat

Fig.9

H=265um

Fig.10

$$\text{Roughness} = \frac{h}{D}$$

Fig.11

$$\text{Evenness} = 1/(\frac{s1 + s2}{A} * 100)$$

Fig.12

$$\text{Uniformity} = \frac{1}{\left| a1 \text{ Contrast ratio} - a2 \text{ Contrast ratio} \right|}$$

Fig.13a

Fig.13b

Fig.13c

Fig.13d

## INTERNATIONAL SEARCH REPORT

| International application No. |
| --- |
| **PCT/KR2024/016005** |

| A. | CLASSIFICATION OF SUBJECT MATTER |
| --- | --- |

**A61L 27/56**(2006.01)i; **A61L 27/58**(2006.01)i; **A61L 27/18**(2006.01)i

According to International Patent Classification (IPC) or to both national classification and IPC

| B. | FIELDS SEARCHED |
| --- | --- |

Minimum documentation searched (classification system followed by classification symbols)

A61L 27/56(2006.01); A61K 45/00(2006.01); D01D 5/00(2006.01); D01D 5/04(2006.01); D04H 1/728(2012.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 다공성 멤브레인(porous membrane), 돌출부(protrusion), 거칠기 (roughness), 나노섬유(nanofiber), 방사(spinning), 전해질(electrolyte)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | KR 10-1965395 B1 (PARK, Jong Su) 04 April 2019 (2019-04-04)<br>See paragraphs [0001] and [0037]-[0087]; and figures 1-8. | 1,2 |
| A | | 3-5 |
| X | KR 10-1602356 B1 (INDUSTRIAL COOPERATION FOUNDATION JEONBUK NATIONAL UNIVERSITY et al.) 11 March 2016 (2016-03-11)<br>See paragraphs [0001] and [0013]-[0045]; and figures 1-7. | 3-5 |
| A | KR 10-2130119 B1 (INDUSTRY-ACADEMIC COOPERATION FOUNDATION, YONSEI UNIVERSITY) 03 July 2020 (2020-07-03)<br>See claim 1. | 1-5 |
| A | KR 10-1691039 B1 (AGENCY FOR DEFENSE DEVELOPMENT) 29 December 2016 (2016-12-29)<br>See claim 1. | 1-5 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **31 January 2025** | **31 January 2025** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/KR2024/016005** |

**C.    DOCUMENTS CONSIDERED TO BE RELEVANT**

| Category* | Citation of document, with indication, where appropriate, of the relevant passages | Relevant to claim No. |
|---|---|---|
| A | JP 2023-513376 A (BAKEL S.R.L.) 30 March 2023 (2023-03-30)<br>    See claim 1. | 1-5 |

Form PCT/ISA/210 (second sheet) (July 2022)

**INTERNATIONAL SEARCH REPORT**
Information on patent family members

International application No.

**PCT/KR2024/016005**

| Patent document cited in search report | | | Publication date (day/month/year) | Patent family member(s) | | | Publication date (day/month/year) |
|---|---|---|---|---|---|---|---|
| KR | 10-1965395 | B1 | 04 April 2019 | None | | | |
| KR | 10-1602356 | B1 | 11 March 2016 | KR | 10-2015-0116492 | A | 16 October 2015 |
| KR | 10-2130119 | B1 | 03 July 2020 | KR | 10-2020-0025028 | A | 10 March 2020 |
| KR | 10-1691039 | B1 | 29 December 2016 | KR | 10-2016-0139264 | A | 07 December 2016 |
| JP | 2023-513376 | A | 30 March 2023 | CN | 115053023 | A | 13 September 2022 |
| | | | | CN | 115066519 | A | 16 September 2022 |
| | | | | EP | 4103771 | A1 | 21 December 2022 |
| | | | | EP | 4103772 | A1 | 21 December 2022 |
| | | | | JP | 2023-513374 | A | 30 March 2023 |
| | | | | KR | 10-2022-0134025 | A | 05 October 2022 |
| | | | | KR | 10-2022-0134026 | A | 05 October 2022 |
| | | | | US | 2023-0081596 | A1 | 16 March 2023 |
| | | | | US | 2023-0083157 | A1 | 16 March 2023 |
| | | | | WO | 2021-161252 | A1 | 19 August 2021 |
| | | | | WO | 2021-161257 | A1 | 19 August 2021 |

Form PCT/ISA/210 (patent family annex) (July 2022)